# EUROPEAN PATENT SPECIFICATION

(11) **EP 0 879 051 B1**
(45) Date of publication and mention of the grant of the patent: **11.06.2003**
(21) Application number: 96908146.2
(22) Date of filing: 29.03.1996
(51) Int. Cl.: A61K 9/70

(54) **RATE-CONTROLLED TRANSDERMAL ADMINISTRATION OF RISPERIDONE**
TRANSDERMALE VERABREICHUNG VON RISPERIDON MIT KONTROLLIERTER GESCHWINDIGKEIT
ADMINISTRATION TRANSDERMIQUE A VITESSE DE DIFFUSION REGULEE DE RISPERIDONE

(30) Priority: 06.04.1995 EP 95200867
(43) Date of publication of application: 25.11.1998
(73) Proprietor: JANSSEN PHARMACEUTICA N.V., 2340 Beerse (BE)
(72) Inventor: MESENS, Jean, Louis, B-2275 Wechelderzande (BE)
(74) Representative: Quaghebeur, Luc
(86) International application number: EP9601421
(87) International publication number: WO96031201

(56) References cited:
- EP-A- 0 196 132
- EP-A- 0 259 136
- WO-A-91/02527
- WO-A-94/06383
- FR-A- 2 143 564
- GB-A- 2 184 016
- DATABASE CHEMABS CHEMICAL ABSTRACTS SERVICE, COLUMBUS, OHIO, US AN: 120:235292, "Absorption, metabolism, and excretion of risperidone in humans" XP002008846

## Description

This invention relates to a medical patch for the rate-controlled transdermal administration of risperidone and to a method of treating a subject by administering risperidone thereto with said medical patch.

Risperidone is generic to 3-[2-[4-(6-fluoro-1,2-benzisoxazol-3-yl)-1-piperidinyl]ethyl]-6,7,8,9-tetrahydro-2-methyl-4H-pyrido[1,2-a]pyrimidin-4-one. The preparation and pharmacological activity thereof are described in U.S. Pat. No. 4,804,663 [corresponding to EP-0,196,132 (1984)]. Various conventional pharmaceutical dosage forms, including tablets, capsules, drops, suppositories, oral solutions and injectable solutions are exemplified therein. In practice, risperidone will normally be administered as the base in a tablet or in a buffered, oral or intramuscular solution for the purposes of producing an antipsychotic effect or alleviating behavioral disturbances associated with neurodegenerative disorders. For a number of reasons, it is desirable to administer risperidone in a rate-controlled manner using a non-invasive route, in particular using transdermal delivery.

EP-0,259,136 describes a transdermal delivery device having a rate controlling adhesive.
GB 2,184,016 discloses a transdermal device for the administration of medicaments.
FR 2,143,564 concerns a transdermal delivery system for the systemic administration of drugs.
WO 94/06383 relates to the transdermal controlled delivery of pharmaceuticals at various dosage rates and processes.

The application of transdermal drug delivery technology to the administration of a wide variety of drugs has been proposed and various systems for accomplishing this are disclosed in numerous technical journals, handbooks and patents. These systems can deliver controlled amounts of drugs to patients for extended periods of time ranging in duration from several hours to several days. So far there are no patents nor is there any other prior art that describes a transdermal delivery system which is intended to deliver risperidone. Nor are there data on skin permeability or therapeutic transdermal delivery rates of risperidone adequate to design such a system. In addition, risperidone has characteristics which impose a combination of restraints on a transdermal delivery system which have hitherto not been addressed in other systems.

Risperidone is a highly potent drug having a relatively narrow therapeutic index. It may produce undesirable side effects on overdosage, most notably extra pyramidal syndrome (EPS) and to a lesser extent hypotension (due to peripheral alpha-adrenergic activity). For the purpose of producing an antipsychotic effect in a patient the total daily dose of risperidone ranges from about 2 to about 8 mg; for the alleviation of behavioral disturbances associated with neurodegenerative disorders the total daily dose is usually less and typically ranges from about 0.5 to about 2 mg. Inter-individual differences and additional medication may necessitate dose titrating in patients.

Risperidone is metabolized to 9-hydroxyrisperidone which has a pharmacological profile and potency comparable with that of the parent drug risperidone, but which has a longer elimination half-life. Risperidone is distributed to and eliminated from the brain tissues more rapidly than its metabolite 9-hydroxyrisperidone.

In the metabolism of risperidone, debrisoquine-type genetic polymorphism plays a distinct role. As a consequence, humans can be phenotyped as poor, intermediate or extensive metabolizers on the basis of their metabolic ratio. Said metabolic ratio is defined as the ratio of urine recovery of debrisoquine to that of the 4-hydroxymetabolite of debrisoquine over a period of 8 hours after an oral intake of 10 mg debrisoquine. In oriental people more than 99% of the population can be phenotyped as extensive metabolizers and poor metabolizers are rather seldom. In the Caucasian race however only about 90% of the population can be phenotyped as either extensive or intermediate metabolizers. Approximately 10% of the population are poor metabolizers and have deficient amounts of the debrisoquine-hydroxylase enzyme.

The duration of action and the peak plasma levels of active agents (risperidone and 9-hydroxyrisperidone) is very dependent on the debrisoquine metabolic rate of the human subject treated with risperidone. More in particular, in poor metabolizers high transient peak levels of risperidone are likely to be attained when the total daily amount is administered in a single dose. This may give rise to undesired side-effects such as extra pyramidal syndrome (EPS) and hypotension (due to the adrenergic effect).

Further inter-individual differences among humans, as far as the metabolism of risperidone is concerned, are due to the fact that in clinical practice the human subjects to be treated with risperidone will usually receive additional medication, e.g. tranquillizers such as phenothiazines, neuroleptica such as haloperidol, antidepressiva etc., all of which will compete with risperidone for the debrisoquine-hydroxylase enzyme. These drug interactions may seriously affect the metabolism of risperidone, especially in extensive metabolizers, and may result in the occurrence of adverse effects in patients receiving such additional medication.

In general, the rapid distribution of risperidone both in plasma and in brain tissues, indicates that the drug can better be administered in divided doses at regular intervals and can best be administered at a continuous controlled rate so as to avoid excessive peak levels (and the possibility of side-effects) while at the same time maintaining clinically effective drug levels. A rate-controlled transdermal delivery system clearly would offer a practical solution to the above, provided that risperidone can be administered percutaneously.

Further, it would be desirable that the device delivers the drug at a substantially constant rate for at least about 24 hours while at the same time keeping the amount of drug within both the unused and depleted systems to a minimum. Also, the degree to which the system controls the release rate should be relatively high in order to assure that excessive amounts of the drug are not delivered in the event that the skin of a patient has been damaged, has an abnormally high permeability, or the patient is a poor metabolizer. On the other hand, the release rate per unit area of system cannot be selected at such a low level that the onset of an antipsychotic effect is delayed or that adequate dosages are not obtained from reasonably sized systems. In addition to these general design criteria, properties of risperidone such as skin permeability and drug binding in the skin may impose additional conflicting design constraints.

Given risperidone's pharmacological profile, the delivery of risperidone in therapeutic amounts for continuous periods from transdermal systems which rely primarily on skin permeability to control drug input rate, is impossible. It is necessary to deliver the drug from a rate-controlled transdermal system in which the system itself controls the maximum rate at which the drug is delivered through the skin.

Interestingly, it has now been found that risperidone in the presence of a suitable skin permeation enhancer (SPE) can be administered through intact skin at a rate which is sufficient to attain therapeutically meaningful plasma and brain tissue levels. Consequently, the present invention concerns a medical patch for the rate-controlled transdermal administration of risperidone through intact skin for an extended period of time which comprises:
(a) a drug reservoir (1) comprising risperidone (I) in an uncharged form, and a skin permeation enhancer (II) for risperidone in a weight by weight ratio of (I) to (II) from 10:1 to 1:10 which are amounts sufficient to deliver (I) at a therapeutically effective rate for said extended period of time, i.e., and
(b) a drug-impermeable backing (2).

The invention also concerns a use as claimed in claim 19.

There is a relatively wide range of permeability of normal human skin to risperidone and this permeability not only varies from individual to individual and site to site but is also highly dependent on the chemical form of the drug. We have discovered that risperidone in a buffered solution, the form in which risperldone is presented for oral and intramuscular administration, has such a low skin permeability that it is not at all suitable for transdermal delivery, even with the use of skin permeation enhancers. Instead, in order to obtain the delivery rates noted above, the drug should be incorporated in the transdermal therapeutic system in the uncharged form of the base. Consequently, the term risperidone as used hereinafter comprises the base form and other uncharged forms such as its solvates, e.g. its hydrates.

The permeability of normal skin to risperidone base is rather low and an amount of permeation enhancer should be provided in a rate-controlled system sufficient to increase the flux of drug through the skin to a value no less than the flux of drug from the system. Thus, sufficient permeation enhancer should be delivered to increase the permeability of even the most impermeable skin to a value at least equal to that of the patch. This will produce a patch in which at least 50% of the flux is controlled by the patch. It is preferable that the patch be at least 70% controlling and this objective can be obtained if the permeability of skin for the drug is increased to at least 2.4 times the steady state flux from the patch.

Suitable skin penetration enhancers induce a temporary, reversible increase in skin permeability. The skin permeation enhancer (II) to be used in the risperidone patches according to the present invention is selected from the group consisting of fatty acids, monoglyceride esters of such fatty acids, C₈₋₁₈ alkylsaccharides, C₈₋₁₈ acyl carnitines, azone (1-dodecylazacycloheptan-2-one) and C₁₋₁₄ alkyl methylsulfoxides. Fatty acids are saturated and unsaturated C₈₋₂₀ alkanoic acids such as caprylic (C_{8:0}), capric (C_{10:0}), lauric (C_{12:0}), myristic (C_{14:0}), palmitic (C_{16:0}), palmitoleic (C_{16:1}), stearic (C_{18:0}), oleic (C_{18:1}), linoleic (C_{18:2}), linolenic (C_{18:3}), and arachidonic (C_{20:4}) acid. C₈₋₁₈ alkylsaccharides are for example n-octyl-beta-D-glucopyranoside and n-lauryl-beta-D-glycopyranoside. C₁₋₁₄ alkyl methylsulfoxides are for example dimethylsulfoxide and decyl methysulfoxide. Preferred are the saturated C₈₋₁₄ alkanoic acids and the unsaturated C₁₆₋₂₀ alkanoic acids having cis double bonds, especially lauric acid and olcic acid. The skin permeation enhancer (II) should be used in an amount that does not damage the skin of the subject to be treated; in practice this means that the weight-by-weight ratio (I): (II) in the patches ranges from 10:1 to 1:10 and in particular is from about 1:1 to about 1:5 in the case of the especially preferred lauric acid and oleic acid.

Risperidone in the presence of a skin permeation enhancer thus may be administered to the human body via the transdermal route at a therapeurically effective rate for an extended period of time: a rate in the range of 10 to 400 µg/h for a substantial portion of said extended period of time is feasible. Steady-state administration rates obtainable range from about 10-300 µg/h and preferably from about 25-150 µg/h.

When transdermal systems according to this invention are applied to the skin, the drug will be transferred from the system into the skin where it is absorbed into the bloodstream to produce its systemic antipsychotic effect. The skin contains risperidone binding sites which must be saauated before any significant absorption into the bloodstream occurs. The initial saturation of these sites should proceed rapidly in order to provide rapid onset of an antipsychotic effect. Since most transdermal therapeutic systems exhibit an initial transitory, increased release of drug which occurs at a significantly higher rate than the steady-state rate later obtained, inclusion of additional amounts of the drug at the skin contacting surface of the device is not an absolute requirement. The systems described herein should be capable of delivering drug at initial rates which should induce the onset of an antipsychotic effect within from four to eight hours after application.

The skin binding sites are significant in establishing an upper limit on the size of the transdermal therapeutic system and, conversely, the lower limit on the usable delivery rate. The total amount of drug contained in the binding sites is directly proportional to the surface area of the delivery system and is independent of the rate at which the drug is delivered. When such a system is removed the total amount of bound drug must be absorbed by the body before the action of the drug stops. In view of the high potency of risperidone it is preferable that the amount of drug solubilized in the skin be maintained at or below 0.5 to 1 mg level to permit prompt termination of therapy.

A suitable patch according to the present invention delivers risperidone through about 5-100 cm² and preferably about 10-50 cm², especially about 20 cm² of intact skin over an extended period of time, and at a rate within the range of about 0.5 to 20 µg/cm².h and preferably at a rate within the range of approximately 1-5 µg/cm².h. When so delivered it is possible, by appropriate selection of the surface area of the drug delivery device to obtain total drug input rates which provide an adequate range of titration for individual patient needs while maintaining a safe and effective dosage form. A suitable patch according to the present invention comprises sufficient (I) and (II) to allow administration for up to 4 days. A 1 to 3 day regimen is considered preferable.

When a prolonged antipsychotic effect is desired, the depleted patch is removed and a fresh system applied to a new location. Since saturation of the skin binding sites usually occurs at substantially the same rate as absorption of bound drug, blood levels will remain substantially constant.

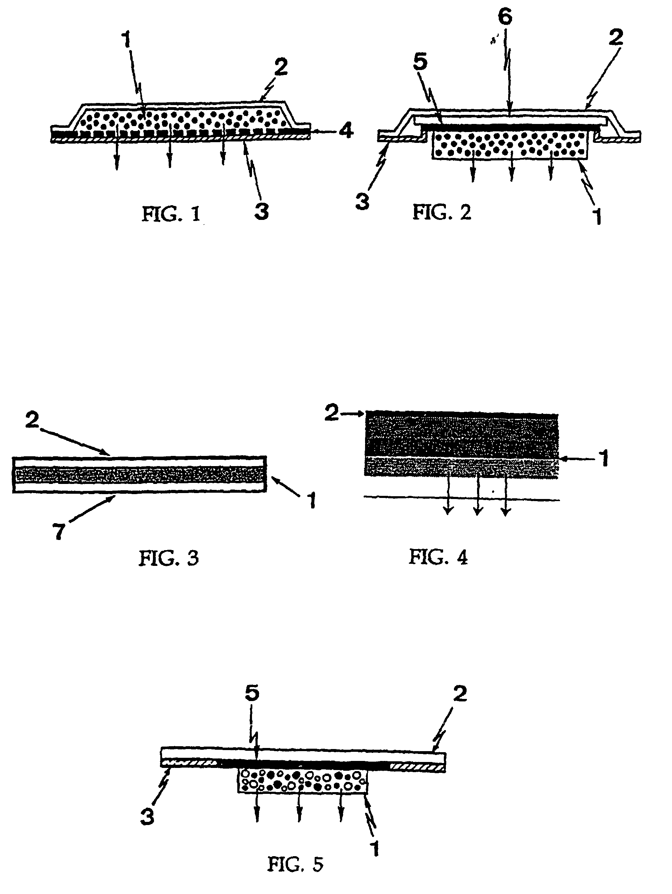

Having described the requirements imposed on transdermal therapeutic systems for administering the base form of risperidone and methods for its transdermal administration, the following description concerns various specific rate-controlled transdermal patch designs. Figures 1 to 5 are all cross-sectional views of such patches.

A first type of rate-controlled transdermal patch is shown in Figure 1: a polymer membrane permeation-controlled patch wherein the drug reservoir (1) is sandwiched between the drug-impermeable backing (2) and a rate-controlling polymeric membrane (4) on the external surface of which a layer (3) of an adhesive polymer is applied. The rate-controlling polymeric membrane (4) has a specific permeability and may be made of a nonporous (homogeneous or heterogeneous) polymeric material or of a porous (semipermeable) membrane.

The drug reservoir (1) may exist in solid, suspension or solution form. Preferably, the drug reservoir (1) contains:
(i) a suspension comprising (I) and (II) dispersed homogeneously in a solid polymer matrix suspended in a liquid medium, [e.g. polisobutylene in silicone fluid] or
(ii) a drug solution comprising (I) and (II) in a releasable solvent [e.g. alkyl alcohols and diols such as ethanol, 1-propanol, 1-butanol, 1-octanol, lauryl alcohol, linolenyl alcohol, and propylene glycol]. The releasable solvents may have skin permeation enhancing properties for risperidone by themselves, e.g. propylene glycol. Therefore, the drug solution comprises in particular propylene glycol as a releasable solvent.

A patch wherein the drug reservoir (1) contains a solution comprising 0.1 to 10% by weight of (I), 0.1 to 10% by weight of (II), and propylene glycol ad 100 % is presently considered the formula of choice for insertion in a transdermal delivery system as shown in Fig. 1.

The drug-impermeable backing (2) is preferably a plastic laminate, e.g. a polyester film laminate or a polyester-polyurethane film (which is air and water permeable). Preferably it is flexible and it may be metalized. In order to obtain a system wherein the flux is controlled by the system itself and not just by the permeability of the skin, the rate-controlling polymeric membrane (4) limits the flux of (I) and (II) from the drug reservoir to a level less than the flux of (I) and (II) through the skin to which it is applied.

In order to minimize the amount of skin permeation enhancer delivered to the patient, it is advantageous that patch has a polymeric membrane (4) which restricts the flux of (I) from the drug reservoir (1) substantially more than the flux of (II) from the drug reservoir (1).

The rate-controlling membrane can be from about 0.5-5 mm thick and preferably about 1-3 mm thick. It can be conveniently made of ethylene-vinyl acetate copolymer (EVA), a polyvinyl acetate or polypropylene. To provide adequate system life, the loading will be from about 5-50 mg/cm² yielding a dry loading of from about 0.01-5 mg/cm².

The adhesive layer (3) comprises a drug-compatible, hypoallergenic pressure-sensitive adhesive polymer and may be disposed in the flow path of (I) and (II) from the drug reservoir (1) to the skin. Silicone adhesive, polyacrylate, polyisobutylene, are considered particularly useful. However, other means for maintaining this system (and the following designs) on the skin can be employed. Such means include a peripheral ring of adhesive outside the path of drug from the system to the skin, in which case the adhesive need not be drug compatible. The use of adhesive overlays or other fastening means such as buckles, belts, and elastic arm bands is also contemplated.

The drug formulation inside the reservoir can be introduced by injection molding, spray coating, microencapsulation and other techniques known in the art. The rate of drug release from the polymer membrane permeation-controlled system should be constant. The release of drug from this type of rate-controlled drug delivery system is controlled by appropriately choosing the partition coefficient and diffusivity of the drug and the thickness and nature of the rate-controlling polymeric membrane (4).

A second type of rate-controlled patch is shown in Figure 2: a polymer matrix dif-fusion-controlled patch wherein the drug reservoir (1) comprises a matrix of a hydro-philic or lipophilic polymer wherein (I) and (II) are homogeneously dispersed, said drug reservoir (1) being mounted onto an occlusive baseplate (5) in a compartment fabricated from the drug-impermeable backing (2), and wherein the adhesive layer (3) is applied along the circumference of the patch to form a strip of adhesive rim surrounding the drug reservoir (1). The medicated polymer is formed by homogeneously dispersing (I), (II) and polymer. It is then shaped into a form having a defined surface area and controlled thickness, which form is then mounted onto the occlusive baseplate (5). At steady state the release is continuous, but not constant as the amount of drug delivered is proportional with the square root of the period of time. In this type of patch, the release of drug is controlled by appropriately choosing the loading dose and the polymer. An absorbent pad (6) can be sandwiched between (2) and (5).

The drug reservoir can also be formulated by directly dispersing the drug in a pressure-sensitive adhesive polymer such as poly(isobutylene)- or a poly(acrylate)-based adhesive polymer. The medicated adhesive polymer is then attached to the drug-impermeable backing to form a single layer or multiple layers of drug reservoir. A patch design of this type is shown in Figure 3: a polymer matrix diffusion-controlled patch wherein the drug reservoir (1) comprises a pressure-sensitive adhesive polymer wherein (I) and (II) are homogeneously dispersed. A strippable backing member (7) or release liner, preferably siliconized, adapted to be removed prior to use is advantageously used to cover the drug reservoir prior to use.

Figure 4 shows a modification of the previous design : a polymer matrix gradient-controlled patch according to claim 1 wherein the drug reservoir (1) comprises multilaminate adhesive layers of a pressure-sensitive adhesive polymer wherein (I) and (II) are dispersed in a proportional manner thus forming a concentration gradient which raises from the skin contacting surface towards the drug-impermeable layer (2). The increasing drug loading level of the layers compensates for the increase in diffusional path and under appropriate circumstances a constant drug release profile can be obtained. Alternatives to this approach consist of varying the polymer solubility of the impregnated drug or varying the particle size distribution of drug crystals in the various laminates of the adhesive matrix.

Figure 5 shows a fourth type of design: a microreservoir partition-controlled patch wherein the drug reservoir (1) comprises a matrix of a hydrophilic or lipophilic polymer wherein many discrete, unleachable, microscopic drug reservoirs comprising (I) and (II) are homogeneously dispersed, said drug reservoir (1) being mounted onto an occlusive baseplate (5) in a compartment fabricated from the drug-impermeable backing (2), and wherein the adhesive layer (3) is applied along the circumference of the patch to form a strip of adhesive rim surrounding the drug reservoir (1). The drug reservoir is formed by first suspending the drug solids in an aqueous solution of a water-miscible drug solubilizer, and then homogeneously dispersing the drug suspension in a lipophilic polymer by high shear mechanical force to form a multitude of unleachable, microscopic drug reservoirs. The thermodynamically unstable dispersion is fixed by immediately crosslinking the polymer chains in situ. In this type of patch, the release of drug can follow either a partition-controlled or matrix diffusion-controlled process depending on the solubilities of the drug in the liquid compartments and in the polymer matrix.

Such a system has the advantage of being easily fabricated, but in the absence of a rate controlling membrane, delivers drug at a rate which is determined primilarily by the permeability of the skin at the site of application on the particular individual. Thus, while this system can be employed to provide drug delivery rates within the ranges described herein, the actual delivery rate cannot be as precisely controlled as would be with the systems described generally in Fig. 1. Suitable materials for fabricating of the contact adhesive/reservoir layer include EVA polymers having up to 18% vinylacetate content and polyisobutylene/mineral oil containing from 15 to 25% high molecular weight polyisobutylene (e.g., an average molecular weight 1,200,000) 20 to 30% low molecular weight polyisobutylene (e.g. average molecular weight 35,000) and balance of light mineral oil having a viscosity at 38°C of approximately 10 mPa.s (centipoise). In addition to the drug, the drug reservoir-contact adhesive layer can also contain additives, permeation enhancers and other materials as are generally known to the art.

The drug reservoirs described above may comprise further ingredients. In order to prevent the growth of micro-organisms such as bacteria, yeasts and fungi in the patches, a preservative agent may be added. Suitable preservatives should be physicochemically stable and effective in the circumstances mentioned above. They comprise benzoic acid, sorbic acid, methylparaben, propylparaben, imidazolidinyl urea (= Germall 115®) and diazolidinyl urea (= Germall II®), phenoxetol, benzyl alcohol, quaternary compounds, e.g. benzylalkonium chloride, and the like. The concentration of the preservatives may range from 0.05% to 1%, particularly from 0.1% to 0.5%, and most particularly is about 0.2%.

The drug reservoir may also be sterilized following art-known procedures. Drug reservoirs may be sterilized by irradiation with gamma rays. Drug solutions can be filtered aseptically and then sterilized by autoclaving.

The patches optionally may include additional ingredients known in the art of formulation such as stabilizers (EDTA), antioxidants (BHT, BHA), solubility enhancers (cyclodextrins), viscosity regulating agents, surfactants (especially non-ionic), hydrating agents (urea), plasticizers (isopropyl myristate) and the like ingredients.

In order for the residual drug in depleted systems to be minimized, we have discovered that the initial concentration of the risperidone in the matrix material should be selected such that it is less than 1 mg/cm². For this reason reservoir systems which permit unit activity to be achieved at low concentrations are presently considered preferable according to our invention.

Several patents describe a wide variety of materials which can be used for fabricating the various layers of the transdermal risperidone delivery systems according to this invention. This invention therefore contemplates the use of materials other than those specifically disclosed herein, including those which may hereafter become known to the art to be capable of performing the necessary functions.

The following examples are intended to illustrate the scope of the present invention in all its aspects; all percentages are by weight unless otherwise noted.

### Experimental Part : In vitro study of the transdermal permeation of risperidone.

An *in vitro* model using a vertical, two-compartment diffusion cell assembly mounted with freshly excised full thickness abdominal skin of hairless rats (Iffa credo) was used in this study. The donor solutions had a volume of 1.5 ml and comprised risperidone, H³-risperidone (1µCi/ml), solvent and permeation enhancer. The receptor solution in all experiments consisted of Hanks Balanced Salt Solution (Gibco BRL) with sodium azide). The skin permeation profile was followed for up to 50 h by sampling the receptor solution and assaying the drug concentration by measuring the radioactivity (Ready Safe, Beckmann-Wallac 1410LKB). The ratio of the cumulated quantities detected in the receptor compartment to the skin area (ng/cm²) were plotted in function of the time and the flux of risperidone through the skin in the presence of various solvents and skin permeation enhancers was deduced from the linear part of the plotted results.

Experiment 1: borate buffer pH 10,0.1 M + risperidone at 40mg/ml
flux : 0.181µg/cm².h
latency period: not determined

Experiment 2 : borate buffer pH 10,0.1 M (900 µl/ml) + ethanol (95%; 100 µl/ml) + risperidone (100 µg/ml)
flux: 0.869 µg/cm².h
latency period: 13.9 h

Experiment 3 : propylene glycol + eucalyptus oil (0%, 1%, 5%) +
risperidone 10 mg/ml
flux (0%): 3.89 µg/cm².h
flux (1%): 4.54 µg/cm².h
flux (5%): 126.74 µg/cm².h
latency period (0%): 14.2 h
latency period (LA %): 15.2 h
latency period (5%): 6.2 h

Experiment 4: propylene glycol + oleic acid (OA) or lauric acid (LA) (0%, 5%) + risperidone 10 mg/ml
flux (0%): 3.89 µg/cm².h
flux (5% OA): 18.9 µg/cm².h
flux (5% LA): 15.39 µg/cm².h
latency period (0%): 14.2 h
latency period (5% OA): 17 h
latency period (5% LA): 4.3 h

From these results one can draw several conclusions
1. Risperidone permeation from a borate buffer with or without ethanol is too low to be of practical value.
2. Risperidone permeation from propylene glycol is decidedly larger than the previous. The very high flux with 5% eucalyptus oil as skin permeation enhancer probably is due to skin damage.
3. Oleic and lauric acid each enhance the transciermal permeation of risperidone from propylene glycol in such a manner that it should be feasible to deliver meaningful doses of risperidone (say from 0.5 to 8 mg/day) in a reasonably sized patch (about 20 cm²).

### Example 1

10 g of risperidone and 50 g of oleic acid is dissolved in 100 ml propylene glycol by stirring. This solution is added to an aqueous acrylate adhesive dispersion while mixing. An adhesive thickener is added and the resulting mixture is stirred until homogenous. Then, the mixture is coated on an impermeable backing such as a polyester film laminate and dried. A release liner (e.g. a siliconized plastic sheet) is laminated to the adhesive layer. The final sheet is die cut to form transdermal devices of about 20 cm² comprising about 20 mg risperidone, each of which is packaged individually.

## Claims

1. A medical patch for the rate-controlled transdermal administration of risperidone through intact skin for an extended period of time which comprises:
(a) a drug reservoir (1) comprising risperidone (I) in an uncharged form, and a skin permeation enhancer (II) for risperidone in a weight by weight ratio of (I) to (II) from 10:1 to 1:10 which are amounts sufficient to deliver (I) at a therapeutically effective rate for said extended period of time, and
(b) a drug-impermeable backing (2).

2. A patch according to claim 1 wherein the skin permeation enhancer (II) is selected from the group consisting of fatty acids, monoglyceride esters of fatty acids, C₈₋₁₈ alkylsaccharides, C₈₋₁₈ acylcarnitines, azone and C₁₋₁₄ alkyl methylsulfoxides.

3. A patch according to claim 2 wherein (I) is delivered through intact skin at a rate in the range of 10 to 400 µg/h for a substantial portion of said extended period of time.

4. A patch according to claim 2 wherein the area of said intact skin is within the range of 5 to 100 cm².

5. A patch according to claim 2 wherein (I) is delivered through intact skin at a rate in the range of 0.5 to 20 µg/h.cm².

6. A patch according to claim 2 comprising sufficient (I) and (II) to allow administration of (I) for up to 4 days.

7. A polymer membrane permeation-controlled patch according to claim 1 wherein the drug reservoir (1) is sandwiched between the drug-impermeable backing (2) and a rate-controlling polymeric membrane (4) on the external surface of which a layer (3) of an adhesive polymer is applied.

8. A patch according to claim 7 wherein the drug reservoir (1) contains
(i) a suspension comprising (I) and (II) dispersed homogeneously in a solid polymer matrix suspended in a liquid medium, or
(ii) a drug solution comprising (I) and (II) in a releasable solvent.

9. A patch according to claim 8 wherein the drug solution comprises propylene glycol as a releasable solvent.

10. A patch according to claim 9 wherein the drug reservoir (1) contains a solution comprising 0.1 to 10% by weight of (I), 0.1 to 10% by weight of (II), and propylene glycol ad 100 %.

11. A patch according to claim 7 wherein the rate-controlling polymeric membrane (4) is either a porous polymeric membrane or a nonporous polymeric membrane.

12. A patch according to claim 11 wherein the rate-controlling polymeric membrane (4) limits the flux of (I) and (II) from the drug reservoir to a level less than the flux of (I) and (II) through the skin to which it is applied.

13. A patch according to claim 11 wherein the polymeric membrane (4) restricts the flux of (I) from the drug reservoir (1) substantially more than the flux of (II) from the drug reservoir (1).

14. A patch according to claim 7 wherein the adhesive layer (3) is disposed in the flow path of (I) and (II) from the drug reservoir (1) to the skin.

15. A polymer matrix diffusion-controlled patch according to claim 1 wherein the drug reservoir (1) comprises a matrix of a hydrophilic or lipophilic polymer wherein (I) and (II) are homogeneously dispersed, said drug reservoir (1) being mounted onto an occlusive baseplate (5) in a compartment fabricated from the drug-impermeable backing (2), and wherein the adhesive layer (3) is applied along the circumference of the patch to form a strip of adhesive rim surrounding the drug reservoir (1).

16. A polymer matrix diffusion-controlled patch according to claim 1 wherein the drug reservoir (1) comprises a pressure-sensitive adhesive polymer wherein (I) and (II) are homogeneously dispersed.

17. A polymer matrix gradient-controlled patch according to claim 1 wherein the drug reservoir (1) comprises multilaminate adhesive layers of apressure-sensitive adhesive polymer wherein (I) and (II) are dispersed in a proportional manner thus forming a concentration gradient which raises from the skin contacting surface towards the drug-impermeable layer (2).

18. A microreservoir partition-controlled parch according to claim 1 wherein the drug reservoir (1) comprises a matrix of a hydrophilic or lipophilic polymer wherein many discrete, unleachable, microscopic drug reservoirs comprising (I) and (II) are homogeneously dispersed, said drug reservoir (1) being mounted onto an occlusive baseplate (5) in a compartment fabricated from the drug-impermeable backing (2), and wherein the adhesive layer (3) is applied along the circumference of the patch to form a strip of adhesive rim surrounding the drug reservoir (1).

19. Use of risperidone for the manufacture of a medical patch as claimed in any one of claims 1 to 18 which administers risperidone through an area of intact skin at a therapeutically effective rate for an extended period of time for inducing and maintaining an antipsychotic effect or for alleviating behavioral disturbances associated with neurodegenerative disorders.

## Patentansprüche

1. Medizinisches Pflaster zur geschwindigkeitskontrollierten transdermalen Verabreichung von Risperidon über intakte Haut über einen längeren Zeitraum, umfassend:
(a) ein Arzneimittelreservoir (1), das Risperidon (I) in ungeladener Form und einen Hautpermeationsverstärker (II) für Risperidon in einem Gewichtsverhältnis (I) zu (II) von 10:1 bis 1:10 enthält, wobei diese Mengen zur Verabreichung von (I) in einer therapeutisch wirksamen Geschwindigkeit über den längeren Zeitraum ausreichen, und
(b) eine arzneimittelundurchlässige Unterlage (2).

2. Pflaster nach Anspruch 1, wobei der Hautpermeationsverstärker (II) aus der aus Fettsäuren, Monoglyceridestern von Fettsäuren, C₈₋₁₈-Alkylsacchariden, C₈₋₁₈-Acylcarnitinen, Azonen und C₁₋₁₄-Alkylmethylsulfoxiden bestehenden Gruppe ausgewählt ist.

3. Pflaster nach Anspruch 2, wobei (I) über einen beträchtlichen Teil des längeren Zeitraums mit einer Geschwindigkeit im Bereich von 10 bis 400 µg/h über intakte Haut verabreicht wird.

4. Pflaster nach Anspruch 2, wobei die Fläche der intakten Haut im Bereich von 5 bis 100 cm² liegt.

5. Pflaster nach Anspruch 2, wobei (I) mit einer Geschwindigkeit im Bereich von 0,5 bis 20 µg/h.cm² über intakte Haut verabreicht wird.

6. Pflaster nach Anspruch 3, enthaltend genügend (I) und (II) für eine Verabreichung von (I) über bis zu 4 Tage.

7. Ein permeationskontrolliertes Pflaster mit Polymermembran nach Anspruch 1, wobei das Arzneimittelreservoir (1) sandwichmäßig zwischen der arzneimittelundurchlässigen Unterlage (2) und einer geschwindigkeitskontrollierenden Polymermembran (4) auf der externen Oberfläche, auf die eine Schicht (3) eines Klebepolymers aufgetragen ist, angeordnet ist.

8. Pflaster nach Anspruch 7, wobei das Arzneimittelreservoir (I)
(i) eine (I) und (II) homogen dispergiert in einer festen Polymermatrix, die in einem flüssigen Medium suspendiert ist, enthaltende Suspension, oder
(ii) eine Arzneimittellösung, die (I) und (II) in einem freisetzbaren Lösungsmittel enthält,
enthält.

9. Pflaster nach Anspruch 8, wobei die Arzneimittellösung Propylenglykol als freisetzbares Lösungsmittel enthält.

10. Pflaster nach Anspruch 9, wobei das Arzneimittelreservoir (1) eine Lösung enthält, die 0,1 bis 10 Gew.-% (I), 0,1 bis 10 Gew.-% (II) und Propylenglykol ad 100% enthält.

11. Pflaster nach Anspruch 7, wobei es sich bei der geschwindigkeitskontrollierenden Polymermembran (4) entweder um eine poröse Polymermembran oder um eine nichtporöse Polymermembran handelt.

12. Pflaster nach Anspruch 11, wobei die geschwindigkeitskontrollierende Polymermembran (4) den Fluß von (I) und (II) aus dem Arzneimittelreservoir auf ein Niveau begrenzt, das unter dem Fluß von (I) und (II) durch die Haut, auf die das Pflaster aufgetragen ist, liegt.

13. Pflaster nach Anspruch 11, wobei die Polymermembran den Fluß von (I) aus dem Arzneimittelreservoir (1) wesentlich stärker eindämmt als den Fluß von (II) aus dem Arzneimittelreservoir (1).

14. Pflaster nach Anspruch 7, wobei die Klebeschicht (3) in der Flußrichtung von (I) und (II) aus dem Arzneimittelreservoir (1) zur Haut angeordnet ist.

15. Diffusionskontrolliertes Pflaster mit Polymermatrix nach Anspruch 1, wobei das Arzneimittelreservoir (1) eine Matrix eines hydrophilen oder lipophilen Polymers umfaßt, in der (I) und (II) homogen dispergiert sind, wobei das Arzneimittelreservoir (1) auf einer okklusiven Grundplatte (5) in einem aus der arzneimittelundurchlässigen Unterlage (2) gefertigten Abteil angebracht ist und wobei die Klebeschicht (3) entlang des Umfangs des Pflasters angebracht ist, so daß ein Streifen eines das Arzneimittelreservoir (1) umgebenden Kleberands gebildet wird.

16. Diffusionskontrolliertes Pflaster mit Polymermatrix nach Anspruch 1, wobei das Arzneimittelreservoir (1) ein Haftklebepolymer enthält, in dem (I) und (II) homogen dispergiert sind.

17. Gradientenkontrolliertes Pflaster mit Polymermatrix nach Anspruch 1, wobei das Arzneimittelreservoir (1) Multilaminatklebeschichten eines Haftklebepolymers umfaßt, in dem (I) und (II) in proportionaler Weise dispergiert sind, so daß ein Konzentrationsgradient ausgebildet wird, der von der mit der Haut in Kontakt stehenden Oberfläche zur arzneimittelundurchlässigen Schicht (2) ansteigt.

18. Aufteilungskontrolliertes Pflaster mit Mikroreservoir nach Anspruch 1, wobei das Arzneimittelreservoir (1) eine Matrix eines hydrophilen oder lipophilen Polymers umfaßt, in der viele diskrete, nicht auslaugbare, mikroskopische Arzneimittelreservoire, die (I) und (II) enthalten, homogen dispergiert sind, wobei das Arzneimittelreservoir (1) auf einer okklusiven Grundplatte (5) in einem aus der arzneimittelundurchlässigen Unterlage (2) gefertigten Abteil angebracht ist und wobei die Klebeschicht (3) entlang des Umfangs des Pflasters angebracht ist, so daß ein Streifen eines das Arzneimittelreservoir (1) umgebenden Kleberands gebildet wird.

## Revendications

1. Timbre médical destiné à l'administration trans-dermique à vitesse contrôlée de rispéridone à travers de la peau intacte pendant une durée prolongée, qui comprend :
(a) un réservoir à médicament (1) comprenant de la rispéridone (I) sous forme non chargée, et un activateur de perméation cutanée (II) pour la rispéridone, dans un rapport pondéral de (I) à (II) allant de 10/1 à 1/10, qui sont les quantités suffisantes pour délivrer (I) à une vitesse efficace sur le plan thérapeutique pendant ladite durée prolongée, et
(b) un support imperméable au médicament (2).

2. Timbre selon la revendication 1, dans lequel l'activateur de perméation cutanée (II) est choisi dans le groupe constitué par les acides gras, les esters de monoglycéride d'acides gras, les alkyl(en C₈₋₁₈)-saccharides, les acyl(en C₈₋₁₈) carnitines, l'azone et les alkyl(en C₁₋₁₄) méthylsulfoxydes.

3. Timbre selon la revendication 2, dans lequel (I) est délivré à travers la peau intacte à une vitesse dans la gamme de 10 à 400 µg/h pendant une partie substantielle de ladite durée prolongée.

4. Timbre selon la revendication 2, dans lequel la surface de ladite peau intacte est dans la gamme de 5 à 100 cm².

5. Timbre selon la revendication 2, dans lequel (I) est délivré à travers la peau intacte à une vitesse dans la gamme de 0,5 à 20 µg/h.cm².

6. Timbre selon la revendication 2 comprenant suffisamment de (I) et de (II) pour permettre une administration de (I) pendant jusqu'à 4 jours.

7. Timbre contrôlé par la perméation à travers une membrane de polymère selon la revendication 1, dans lequel le réservoir à médicament (1) est pris en sandwich entre le support imperméable au médicament (2) et une membrane polymère contrôlant la vitesse (4) sur la surface extérieure de laquelle est appliquée une couche (3) d'un polymère adhésif.

8. Timbre selon la revendication 7, dans lequel le réservoir à médicament (1) contient
(i) une suspension comprenant (I) et (II) dispersés de façon homogène dans une matrice de polymère solide en suspension dans un milieu liquide, ou
(ii) une solution de médicament comprenant (I) et (II) dans un solvant libérable.

9. Timbre selon la revendication 8, dans lequel la solution de médicament comprend du propylèneglycol comme solvant libérable.

10. Timbre selon la revendication 9, dans lequel le réservoir à médicament (1) contient une solution comprenant 0,1 à 10% en poids de (I), 0,1 à 10% en poids de (II) et le complément à 100% de propylèneglycol.

11. Timbre selon la revendication 7, dans lequel la membrane polymère contrôlant la vitesse (4) est soit une membrane polymère poreuse, soit une membrane polymère non poreuse.

12. Timbre selon la revendication 11, dans lequel la membrane polymère contrôlant la vitesse (4) limite le flux de (I) et de (II) depuis le réservoir à médicament à un niveau inférieur au flux de (I) et de (II) à travers la peau sur laquelle il est appliqué.

13. Timbre selon la revendication 11, dans lequel la membrane polymère (4) limite le flux de (I) depuis le réservoir à médicament (1) substantiellement plus que le flux de (II) depuis le réservoir à médicament (1).

14. Timbre selon la revendication 7, dans lequel la couche adhésive (3) est placée sur le trajet d'écoulement de (I) et de (II) allant du réservoir à médicament (1) jusqu'à la peau.

15. Timbre contrôlé par la diffusion à travers une matrice de polymère selon la revendication 1, dans lequel le réservoir à médicament (1) comprend une matrice d'un polymère hydrophile ou lipophile dans laquelle (I) et (II) sont dispersés de façon homogène, ledit réservoir à médicament (1) étant monté sur une plate-forme occlusive (5) dans un compartiment fabriqué à partir du support imperméable au médicament (2), et dans lequel la couche adhésive (3) est appliquée sur la circonférence du timbre pour former une bande de cordon adhésif entourant le réservoir à médicament (1).

16. Timbre contrôlé par la diffusion à travers une matrice de polymère selon la revendication 1, dans lequel le réservoir à médicament (1) comprend un polymère adhésif sensible à la pression dans lequel (I) et (II) sont dispersés de façon homogène.

17. Timbre contrôlé par un gradient à travers une matrice de polymère selon la revendication 1, dans lequel le réservoir à médicament (1) comprend des couches adhésives multicouches d'un polymère adhésif sensible à la pression dans lesquelles (I) et (II) sont dispersés de manière proportionnelle, formant ainsi un gradient de concentration qui s'élève lorsqu'on va de la surface en contact avec la peau jusqu'à la couche imperméable au médicament (2).

18. Timbre contrôlé par le partage entre des micro-réservoirs selon la revendication 1, dans lequel le réservoir à médicament (1) comprend une matrice d'un polymère hydrophile ou lipophile dans laquelle de nombreux réservoirs à médicament microscopiques, discrets, non lixiviables comprenant (I) et (II) sont dispersés de façon homogène, ledit réservoir à médicament (1) étant monté sur une plate-forme occlusive (5) dans un compartiment fabriqué à partir du support imperméable au médicament (2), et dans lequel la couche adhésive (3) est appliquée sur la circonférence du timbre pour former une bande de cordon adhésif entourant le réservoir à médicament (1).
